# EUROPEAN PATENT APPLICATION

(11) **EP 4 691 347 A1**
(43) Date of publication of application: **11.02.2026**
(21) Application number: 24208006.7
(22) Date of filing: 22.10.2024
(51) Int. Cl.: A61B 5/00

(54) **MOBILE ROBOT FOR GAIT ANALYSIS USING ROTARY LIDAR**

(30) Priority: 07.08.2024 KR 20240104992
(71) Applicant: Medical Solution System, Gimhae city, Gyeongsangnam-do 50834 (KR)
(72) Inventor: KANG, Minsoo, 47507 Busan (KR); Seo, Joon Mo, 48270 Busan (KR)
(74) Representative: M. Zardi & Co S.A.

(57) **Abstract**

Proposed is a mobile robot for gait analysis using a rotary LiDAR which includes a main body, a driving unit for moving the main body, a front LiDAR sensor unit mounted on the main body to scan a front side, a controller for controlling the driving unit to follow a target to be measured with a preset distance maintained, by using scanning information of the front LiDAR sensor unit, a gait measurement LiDAR sensor unit being installed to protrude forward from a side of the main body and being composed of at least two rotary LiDARs of measuring the foot movements of the target to be measured, and a gait analysis unit for analyzing a gait of the target to be measured, by using the foot movements measured by the gait measurement LiDAR sensor unit.

## Description

### CROSS REFERENCE TO RELATED APPLICATION

The present application claims priority to Korean Patent Application No. 10-2024-0104992, filed on Aug. 07, 2024, the entire contents of which are incorporated herein for all purposes by this reference.

### BACKGROUND

### Technical Field

The present disclosure relates to a mobile robot for gait analysis and, more particularly, to a mobile robot for gait analysis using a rotary LiDAR, which is capable of analyzing the gait of a target to be measured.

### Description of the Related Art

The description in this section merely provides background information on an exemplary embodiment of the present disclosure and does not constitute the prior art.

Gait analysis is to find out the singularity of the walking pattern, and performs a comprehensive evaluation by measuring various aspects of gait. More specifically, it may include kinematic analysis, dynamic electromyography, and energy expenditure measurement. This gait analysis can be applied to identify the posture or movement characteristics of people with lower extremity lesions and spinal cord misalignments, or to help athletes run more efficiently.

Currently, gait analysis in rehabilitation medicine measures and analyzes gait by using expensive load-measuring mattresses or high-speed cameras. However, this type of gait measurement has problems such as requiring various sensors to be attached to the target's body, being expensive, and taking a long time to prepare for the measurement. In addition, there are limitations in analyzing an everyday gait since the analysis is performed in a controlled environment such as a laboratory.

Recently, as people spend more time sitting, scoliosis, pelvic distortion, and flat feet have become more common, and these problems affect gait. In particular, for children and adolescents, it is important to detect and correct the wrong posture early and ensure the development of good walking habits.

Although the need for gait measurement and analysis is increasing, conventional gait analysis methods are not easily accessible due to the cost and limitations. Therefore, it is necessary to develop a technology that can analyze gait more simply and at a lower cost.

Meanwhile, as prior art related to the present disclosure, a Korean patent has been disclosed (Patent No. 10-1895399, "Gait analysis and correction apparatus base of information and communications technology", Registration Date: August 30, 2018).

The related art technology described above is technical information that the inventor possessed for deriving the present disclosure or acquired in the process of deriving the present disclosure and cannot necessarily be said to be known to the general public before filing the application for the present disclosure.

### SUMMARY

The present disclosure is proposed to solve the above-mentioned problems of the previously proposed methods, and aims to provide a mobile robot for gait analysis using a rotary LiDAR, which performs gait analysis simply and easily without attaching a sensor to the body of the target to be measured and continuously analyzes gait without restrictions on places since tracking and measuring the target to be measured, by measuring the foot movements of the target to be measured and by analyzing the gait using a gait measurement LiDAR sensor unit including at least two rotary LiDARs which are installed to protrude forward from the side of the main body, while following the target to be measured with a preset distance maintained using the scanning information of a front LiDAR sensor unit mounted on the main body.

In addition, another objective of the present disclosure is to provide a mobile robot for gait analysis using a rotary LiDAR, which detects the same object at the same time by allowing two rotary LiDARs to rotate in a direction different from each other and thus accurately tracks the foot movements of the fast-walking target to be measured, by including a left LiDAR sensor unit where a rotary LiDAR is installed to protrude forward from the left side of the main body in order to rotate in a first direction and a right LiDAR sensor unit where a rotary LiDAR is installed upside down to protrude forward from the right side of the main body in order to rotate in a second direction opposite to the first direction.

However, the technical task to be achieved by the present disclosure is not limited to the technical task described above, other technical tasks may exist, and even if not explicitly mentioned, it also includes purposes or effects that can be identified from the means of solving the tasks or the forms of implementation.

A mobile robot for gait analysis using a rotary LiDAR according to the characteristics of the present disclosure for achieving the objectives includes a main body, a driving unit for moving the main body, a front LiDAR sensor unit mounted on the main body to scan a front side, a controller for controlling the driving unit to follow a target to be measured with a preset distance maintained, by using scanning information of the front LiDAR sensor unit, a gait measurement LiDAR sensor unit being installed to protrude forward from a side of the main body and being composed of at least two rotary LiDARs of measuring the foot movements of the target to be measured, and a gait analysis unit for analyzing a gait of the target to be measured, by using the foot movements measured by the gait measurement LiDAR sensor unit, wherein the gait measurement LiDAR sensor unit includes a left LiDAR sensor unit where a rotary LiDAR is installed to protrude forward from a left side of the main body in order to rotate in a first direction and a right LiDAR sensor unit where a rotary LiDAR is installed to protrude forward from a right side of the main body in order to rotate in a second direction opposite to the first direction.

Preferably, the right LiDAR sensor unit may allow the rotary LiDAR to be installed upside down to rotate in the second direction.

Preferably, the controller may control the driving unit such that a distance from the left LiDAR sensor unit to the target to be measured is the same as a distance from the right LiDAR sensor unit to the target to be measured.

Preferably, the front LiDAR sensor unit may be installed at a torso height of the target to be measured.

Preferably, the gait measurement LiDAR sensor unit may measure the foot movements, including changes in position, direction, and shape of the left and right feet, by scanning a plane at a foot height of the target to be measured.

More preferably, each of the left LiDAR sensor unit and the right LiDAR sensor unit may include a first LiDAR sensor unit for collecting a first scanning data by scanning the plane at the foot height of the target to be measured, and a second LiDAR sensor unit for collecting a second scanning data by scanning a plane at a knee height of the target to be measured.

More preferably, the gait analysis unit may analyze at least one selected from a group consisting of body distortion and gait instability when the target to be measured walks, by combining foot position, knee position, and torso position of the target to be measured.

According to the mobile robot for gait analysis using the rotary LiDAR proposed in the present disclosure, it is possible to perform gait analysis simply and easily without attaching a sensor to the body of the target to be measured and continuously analyze gait without restrictions on places since tracking and measuring the target to be measured, by measuring the foot movements of the target to be measured and by analyzing the gait using a gait measurement LiDAR sensor unit including at least two rotary LiDARs which are installed to protrude forward from the side of the main body, while following the target to be measured with a preset distance maintained by using the scanning information of a front LiDAR sensor unit mounted on the main body.

In addition, according to the mobile robot for gait analysis using the rotary LiDAR, it is possible to detect the same object at the same time by allowing two rotary LiDARs to rotate in a direction different from each other and thus to accurately track the foot movements of the fast-walking target to be measured, by including a left LiDAR sensor unit where a rotary LiDAR is installed to protrude forward from the left side of the main body in order to rotate in a first direction and a right LiDAR sensor unit where a rotary LiDAR is installed upside down to protrude forward from the right side of the main body in order to rotate in a second direction opposite to the first direction.

In addition, various and beneficial advantages and effects of the present disclosure are not limited to the content described above, and may be more easily understood in the process of explaining specific exemplary embodiments of the present disclosure.

### BRIEF DESCRIPTION OF THE DRAWINGS

FIG. 1 is a view showing a configuration of a mobile robot for gait analysis using a rotary LiDAR according to an exemplary embodiment of the present disclosure.
FIG. 2 is a view showing an example of the overall appearance of a mobile robot for gait analysis using a rotary LiDAR according to an exemplary embodiment of the present disclosure.
FIG. 3 is a top view of a mobile robot for gait analysis using a rotary LiDAR according to an exemplary embodiment of the present disclosure.
FIG. 4 is a view showing that a mobile robot for gait analysis using a rotary LiDAR moves and scans the gait of a target to be measured according to an exemplary embodiment of the present disclosure.
FIG. 5 is a view showing a target detection when rotary LiDARs rotating in the same direction are applied.
FIG. 6 is a view showing a target detection when rotary LiDARs rotating in different directions are applied in a mobile robot for gait analysis using a rotary LiDAR according to an exemplary embodiment of the present disclosure.
FIG. 7 is a view showing an example where a gait measurement LiDAR sensor unit of a mobile robot for gait analysis using a rotary LiDAR includes a first LiDAR sensor unit and a second LiDAR senor unit according to an exemplary embodiment of the present disclosure.

### DETAILED DESCRIPTION OF THE DISCLOSURE

With reference to the attached drawings below, exemplary embodiments of the present disclosure will be described in detail so that those of ordinary skill in the technical field to which the present disclosure belongs may easily implement the present disclosure. However, the present disclosure may be implemented in various forms, and is not limited to the exemplary embodiments described herein. Also, in order to clearly describe the present disclosure, parts irrelevant to the description are omitted in the drawings, and similar parts are given similar reference numerals throughout the specification.

Throughout the specification, when an element is "connected" to another element, it includes not only the case where it is "directly connected" but also the case where it is "indirectly connected" to another element with any element interposed in between. In addition, terms such as "include" "provided" or "have" described below should be construed as trying to specify that a feature, number, step, motion, component, part or combination thereof exists in the specification, and it should be understood that the existence or additional possibilities of one or more other features, numbers, steps, motions, components, parts, or combinations thereof are not excluded in advance. In addition, singular expressions used in the present disclosure include plural expressions unless the context clearly indicates otherwise.

In addition, each configuration, process, procedure, method, and the like included in each exemplary embodiment of the present disclosure may be shared within a range that does not technically contradict each other.

In addition, terms such as "...unit", "...device", "...module", and the like used in the specification refer to a unit that processes at least one function or operation, which may be implemented as hardware or software or a combination of hardware and software.

In addition, some of the operations or functions described as being performed by the terminal, apparatus, or device in the present disclosure may be performed by a server connected to the terminal, apparatus, or device. Likewise, some of the operations or functions described as being performed by the server may also be performed by the terminal, apparatus, or device connected to the server.

In particular, a means for executing a system according to each exemplary embodiment of the present disclosure may be an application or a web server, and a terminal as the means that reads a recording medium in which the application or web server is recorded may include not only general PCs such as desktops and laptops, but also mobile terminals such as smartphones and tablet PCs.

The following exemplary embodiments are detailed explanations for assisting in the understanding of the present disclosure and do not limit the scope of the present disclosure. Therefore, inventions performing the same function at the same scope as the present disclosure will also fall within the scope of rights of the present disclosure.

Hereinafter, exemplary embodiments of the present disclosure will be described in detail with reference to the accompanying drawings.

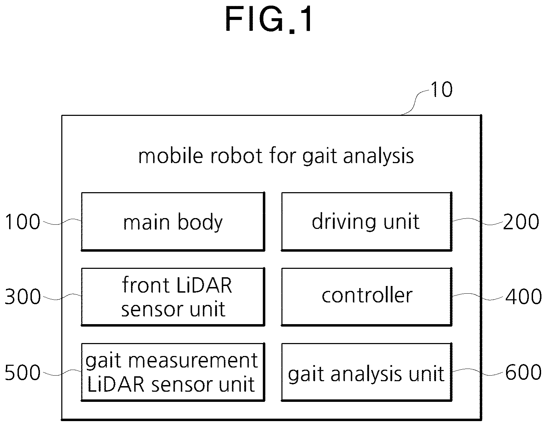

FIG. 1 is a view showing a configuration of a mobile robot 10 for gait analysis using a rotary LiDAR according to an exemplary embodiment of the present disclosure. As shown in FIG. 1, the mobile robot 10 for gait analysis using the rotary LiDAR according to an exemplary embodiment of the present disclosure may include a main body 100, a driving unit 200, a front LiDAR sensor unit 300, a controller 400, a gait measurement LiDAR sensor unit 500, and a gait analysis unit 600.

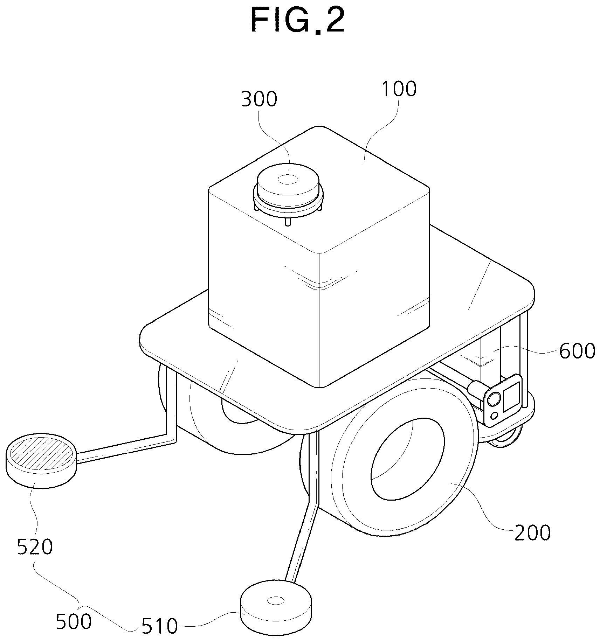

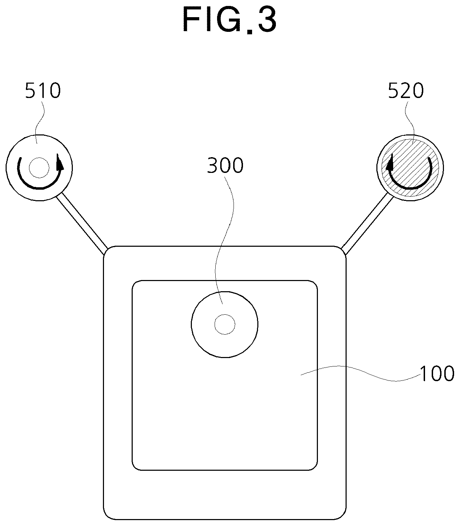

FIG. 2 is a view showing an example of the overall appearance of a mobile robot 10 for gait analysis using a rotary LiDAR according to an exemplary embodiment of the present disclosure, and FIG. 3 is a top view of a mobile robot 10 for gait analysis using a rotary LiDAR according to an exemplary embodiment of the present disclosure. As shown in FIGS. 2 and 3, the gait measurement LiDAR sensor unit 500 of the mobile robot 10 for gait analysis using the rotary LiDAR according to an exemplary embodiment of the present disclosure may be configured to include a left LiDAR sensor unit 510 and a right LiDAR sensor unit 520.

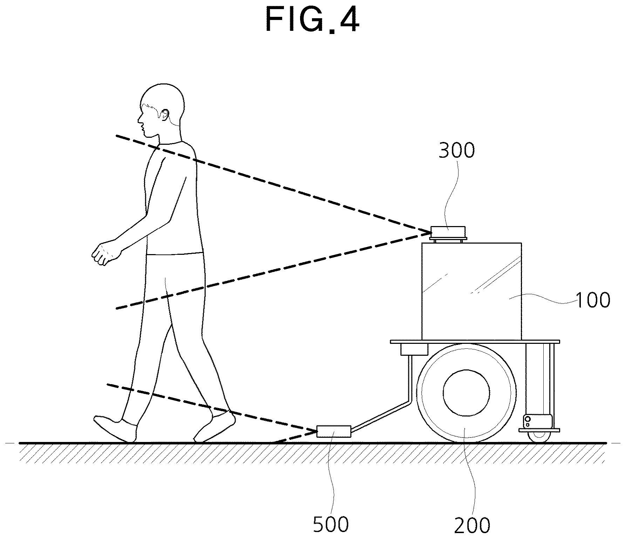

FIG. 4 is a view showing that a mobile robot 10 for gait analysis using a rotary LiDAR moves and scans the gait of a target to be measured according to an exemplary embodiment of the present disclosure. As shown in FIGS. 2 to 4, the mobile robot 10 for gait analysis using the rotary LiDAR according to an exemplary embodiment of the present disclosure may measure the foot movements of the target to be measured and analyze the gait by using the gait measurement LiDAR sensor unit 500 including at least two rotary LiDARs which are installed to protrude forward from the side of the main body 100, while following the target to be measured with a preset distance maintained by using the scanning information of the front LiDAR sensor unit 300 mounted on the main body 100. Therefore, it may be possible to perform gait analysis simply and easily without attaching a sensor to the body of the target to be measured and continuously analyze gait without restrictions on places since tracking and measuring the target to be measured.

In addition, as shown in FIG. 3, it may be possible to detect the same object at the same time by allowing two rotary LiDARs to rotate in a direction different from each other and thus to accurately track the foot movements of the fast-walking target to be measured, by including a left LiDAR sensor unit 510 where a rotary LiDAR is installed to protrude forward from the left side of the main body 100 in order to rotate in a first direction and a right LiDAR sensor unit 520 where a rotary LiDAR is installed upside down to protrude forward from the right side of the main body 100 in order to rotate in a second direction opposite to the first direction.

A LiDAR sensor may be a device that identifies the location and shape of an object through laser scanning and may be applied to aircraft and autonomous vehicles. Using the LiDAR sensor, gait analysis may be performed by accurately measuring footprints, including the position, shape, direction, and form of the foot of the target to be measured.

The LiDAR sensor may be composed of a light-emitting sensor that outputs light and a light-receiving sensor that receives reflected light as input. There may be a fixed type and a rotary type in the LiDAR sensors, and the fixed type may be in the form of a line sensor with one light-emitting sensor and one light-receiving fixed, where multiple sensor channels should be used to scan space. Therefore, the cost may be high, energy efficiency may be low, and data capacity may be large, resulting in an expensive system overall. On the other hand, the rotary type may perform 360-degree two-dimensional scanning as the line sensor rotates. The rotary LiDAR may scan the space at an angular speed over time according to the rotation speed, and may be capable of scanning an area with a single line sensor, making it inexpensive and efficient. However, there are disadvantages that the mechanical rotation causes vibration as well as the movements faster than the rotation speed cannot be detected as sampling is performed according to the rotation speed.

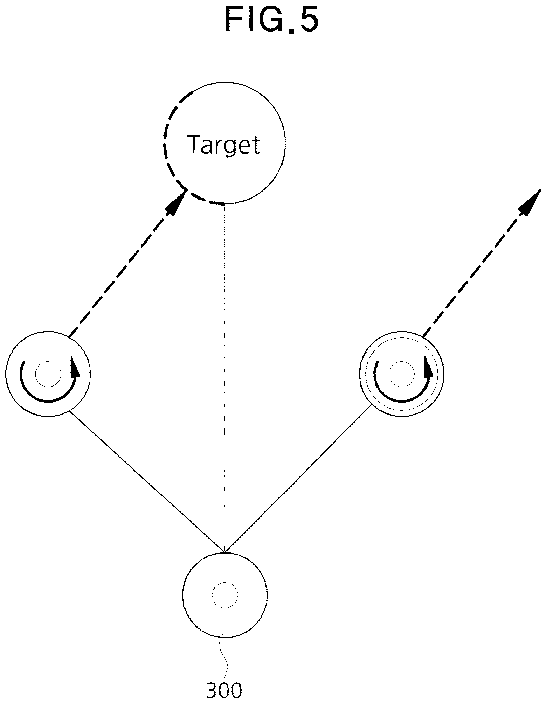

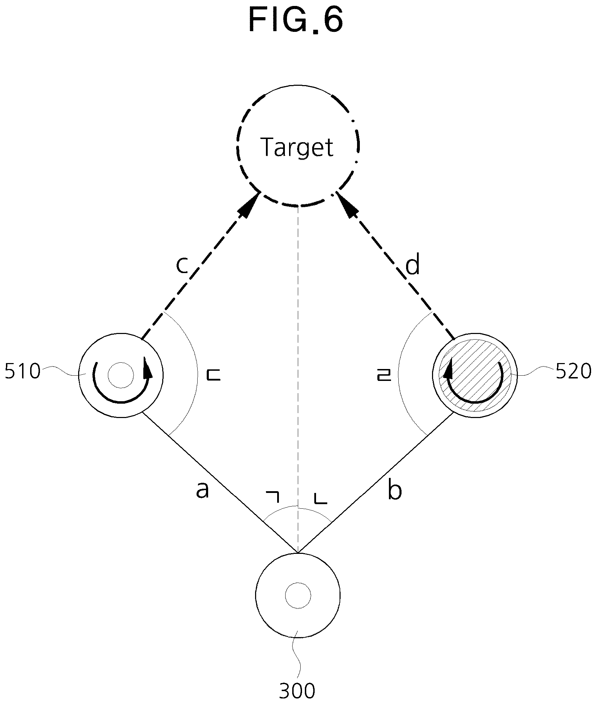

FIG. 5 is a view showing a target detection when rotary LiDARs rotating in the same direction are applied, and FIG. 6 is a view showing a target detection when rotary LiDARs rotating in different directions are applied in a mobile robot 10 for gait analysis using a rotary LiDAR according to an exemplary embodiment of the present disclosure. The rotary LiDAR in the form of a 2D line sensor may be always rotated in one direction, and it may be typical to rotate in a counterclockwise direction. In this case, when the two rotary LiDARs rotate in the same counterclockwise direction as shown in FIG. 5, the measurement directions of the line sensors of the two rotary LiDAR are the same, so the positions of the feet of the target to be measured may be scanned with a time difference. That is, when the rotary LiDAR on the left side scans the target to be measured, the rotary LiDAR on the right side may scan another place such that only one rotary LiDAR scans the foot movements of the target to be measured at a specific time. Therefore, when the target to be measured moves quickly during gait measurement, it may not be possible to accurately track the footprints.

On the other hand, when the two rotary LiDARs are installed to rotate in opposite directions from each other as shown in FIG. 6, the measurement directions of the line sensors of the two rotary LiDARs may be simultaneously directed toward the target to be measured. That is, it may be possible to accurately detect the foot movements of the fast-moving target to be measured since the right LiDAR sensor unit 520 simultaneously scans the target to be measured when the left LiDAR sensor unit 510 scans the target to be measured at a specific time.

In addition, the recognition rate may decrease because of the characteristics of the LiDAR as moving far away from the measurement reference point, so there may be a problem of recognition when the target to be measured is more than 2m away from the LiDAR sensor, in particular, in the case of a footprint when the size of the foot is small. The mobile robot 10 for gait analysis using the rotary LiDAR according to an exemplary embodiment of the present disclosure may be configured as a mobile body and may collect and store a path and a footprint measurement result while following the target to be measured with the mounted gait measurement LiDAR sensor unit 500. Therefore, when using the mobile robot 10 for gait analysis using the rotary LiDAR according to an exemplary embodiment of the present disclosure, it may be possible to continuously track and analyze gait in various environments away from a predetermined place.

Hereinafter, each configuration of the mobile robot 10 for gait analysis using the rotary LiDAR according to an exemplary embodiment of the present disclosure will be described in detail with reference to FIGS. 1 to 6.

The main body 100 may form the body of the mobile robot 10 for gait analysis using the rotary LiDAR according to an exemplary embodiment of the present disclosure and may have various configurations embedded therein, such as the controller 400, the gait analysis unit 600, and the like. In FIG. 2, the main body 100 may be composed of a plate and a box, but is not limited thereto, and the shape of the main body 100 may vary.

The driving unit 200 may move the main body 100. That is, as shown in FIG. 2, the driving unit 200 may be composed of at least one wheel or the like disposed below the main body 100 so that the mobile robot 10 for gait analysis using the rotary LiDAR is configured to be mobile according to an exemplary embodiment of the present disclosure.

The front LiDAR sensor unit 300 may be mounted on the main body 100 to scan the front side. More specifically, the front LiDAR sensor unit 300 may be installed at the torso height of the target to be measured. That is, as shown in FIG. 4, the front LiDAR sensor unit 300 may scan the torso height of the target to be measured going ahead so that the distance to the target to be measured remains constant. In addition, the front LiDAR sensor unit 300 may be installed on the upper side of the main body 100 to scan a plane at the torso height between the knees and shoulders of the target to be measured, thereby detecting the torso position of the target to be measured and the direction in which the torso faces.

The controller 400 may control the driving unit 200 to follow the target to be measured with a preset distance maintained by using the scanning information of the front LiDAR sensor unit 300. That is, the controller 400 may identify the information of the target to be measured in real time using the position and direction of the torso of the target measured in the front LiDAR sensor unit 300, and accordingly control the driving unit 200 to move in an appropriate speed, direction, and the like so that the mobile robot 10 for gait analysis using the rotary LiDAR according to an exemplary embodiment of the present disclosure maintains a certain distance from the target to be measured.

The gait measurement LiDAR sensor unit 500 may be installed to protrude forward from the side of the main body 100 and may include at least two rotary LiDARs measuring the foot movements of the target to be measured. More specifically, the gait measurement LiDAR sensor unit 500 may measure the foot movements, including changes in the positions, directions, and shapes of the left and right feet by scanning a plane at the foot height of the target to be measured. Herein, the laser scanning height of the gait measurement LiDAR sensor unit 500 may be approximately 3 to 10 cm above the ground and, more specifically, may be 5 cm in height. The gait measurement LiDAR sensor unit 500 may track and observe the footprints of the target to be measured by scanning with laser beams the plane parallel to the ground at a height of 3-10 cm from the ground and generating the scanning data.

In addition, the gait measurement LiDAR sensor unit 500 may include a left LiDAR sensor unit 510 where the rotary LiDAR is installed to protrude forward from the left side of the main body 100 in order to rotate in the first direction, and a right LiDAR sensor unit 520 where the rotary LiDAR is installed to protrude forward from the right side of the main body 100 in order to rotate in the second direction opposite to the first direction.

As shown in FIGS. 3 and 6, the mobile robot 10 for gait analysis using the rotary LiDAR according to an exemplary embodiment of the present disclosure may measure the left and right feet of a walking target to be measured with the left LiDAR sensor unit 510 and the right LiDAR sensor unit 520 installed to protrude in a "Y" shape. In this case, "a" and "b" in FIG. 6 may be distances at which the rotary LiDARs are respectively installed to protrude forward from the main body 100 of the mobile robot 10 for gait analysis, and "a" and "b" may be designed to be the same in the mobile robot 10 for gait analysis.

In addition, the controller 400 may control the driving unit 200 such that the distance from the left LiDAR sensor unit 510 to the target to be measured is the same as the distance from the right LiDAR sensor to the target to be measured. That is, the controller 400 may control the driving unit 200 such that the lengths of "c" and "d" are the same as shown in FIG. 6. In this way, "a" and "b" may be designed to be the same and the driving unit 200 may be controlled so that "c" and "d" become the same, so " 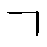 " and " 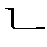 ", and " 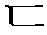 " and " 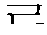 " may respectively have the same angle centered on a line segment connecting the front LiDAR sensor unit 300 and the target to be measured.

Meanwhile, the two LiDAR sensor units 510, 520 may be installed to rotate in opposite directions from each other and, more specifically, the right LiDAR sensor unit 520 may be installed to rotate in the second direction by reversing the rotary LiDAR. Accordingly, since the left LiDAR sensor unit 510 rotates in the first direction (a counterclockwise direction in FIG. 6) and the right LiDAR sensor unit 520 rotates in the second direction (a clockwise direction in FIG. 6), the movements of both feet of the target to be measured may be scanned simultaneously at a specific time. Through this configuration, it may be possible to minimize the possibility of measurement failure caused by interference of other objects, overlapping of the two feet, fast foot movements of the target to be measured, and the like and to accurately measure the position, direction, shape, and the like of the feet.

The gait analysis unit 600 may analyze the gait of the target to be measured by using the foot movements measured in the gait measurement LiDAR sensor unit 500. More specifically, the gait analysis unit 600 may analyze the gait of the target to be measured by comprehensively analyzing the movements of the left foot and the right foot measured by the left LiDAR sensor unit 510 and the right LiDAR sensor unit 520, and the movement path and movement speed of the mobile robot 10 for gait analysis. In FIG. 2, the gait analysis unit 600 may be mounted behind the driving unit 200, but the location of the gait analysis unit 600 may vary, such as being embedded in the main body 100. Depending on exemplary embodiments, the function of the gait analysis unit 600 may be performed in an electronic device connected through a network to the mobile robot 10 for gait analysis.

In addition, the gait analysis unit 600 may analyze at least one selected from the group consisting of a stride length, the number of steps, and a walking speed. First, the gait analysis unit 600 may receive the scanning data generated by the gait measurement LiDAR sensor unit 500 and may recognize the foot position of the target to be measured from the scanning data. Using the recognized foot position, the gait analysis unit 600 may analyze at least one selected from the group consisting of the stride length, the number of steps, and the walking speed. In addition, the left and right gait balance may be analyzed using the positions of the left foot and right foot and each step distance of the left foot and right foot. Depending on exemplary embodiments, the gait analysis unit 600 may recognize the angle of the foot of the target to be measured from the scanning data.

Meanwhile, the gait analysis unit 600 may predict the type of disease from the gait analysis result by using the artificial intelligence model that has been trained completely by inputting the stride length, the number of steps, the walking speed, the left and right gait balance, and the foot angle and outputting the type of disease. That is, the gait analysis results for the target to be measured by the gait analysis unit 600 such as the stride length, the number of steps, the walking speed, the left and right gait balance, and the foot angle may be inputted into the artificial intelligence model, and the types of predicted diseases such as musculoskeletal diseases, vestibular abnormalities, Parkinson's disease, and dementia may be obtained as an output. These output values may be provided to the client terminal of the medical staff to help the medical staff make decisions.

Herein, the learning algorithm of the artificial intelligence model may be any one of a random forest, a convolutional neural network (CNN), a recurrent neural network (RNN), and a transformer, and an ensemble algorithm that combines two or more algorithms may be used.

Depending on exemplary embodiments, transfer learning may be used. Transfer learning may be to reuse a pre-trained model for a new problem. Since a pre-trained model is used, there may be an advantage of being able to train a deep neural network with relatively little data. In addition, it may be useful because most real-world problems typically do not have millions of labeled data to train complex models. Using such transfer learning, the present disclosure may be possible to predict the type of disease with greater detail and accuracy by generating an artificial intelligence model for each group for the target to be measured grouped by gender, age, and the like from an artificial intelligence model pre-trained with large amounts of data.

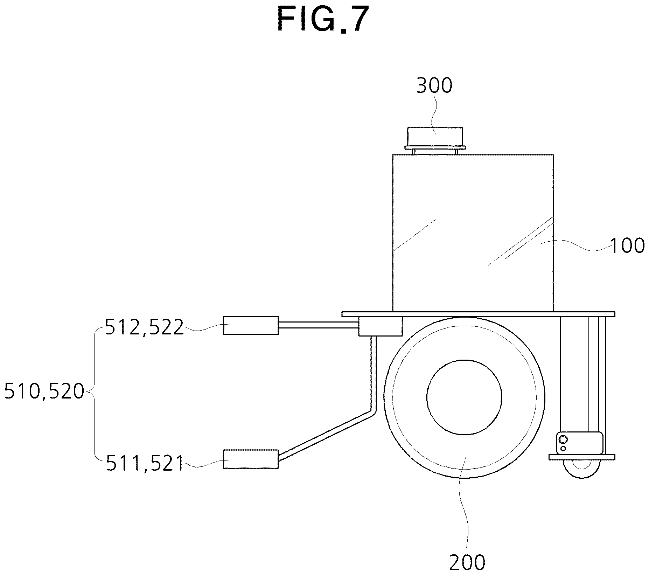

FIG. 7 is a view showing an example where a gait measurement LiDAR sensor unit 500 of a mobile robot 10 for gait analysis using a rotary LiDAR includes first LiDAR sensor units 511, 521 and second LiDAR sensor units 512, 522 according to an exemplary embodiment of the present disclosure. As shown in FIG. 7, the left LiDAR sensor unit 510 and the right LiDAR sensor unit 520 of the mobile robot 10 for gait analysis using the rotary LiDAR according to an exemplary embodiment of the present disclosure may include first LiDAR sensor units 511, 521 that scan a plane at the foot height of the target to be measured and collect a first scanning data and second LiDAR sensor units 512, 522 that scan a plane at the knee height of the target to be measured and collect a second scanning data. The gait analysis unit 600 may analyze at least one selected from the group consisting of body distortion and walking instability when the target to be measured walks, by combining the foot position, knee position, and torso position of the target to be measured.

That is, the left LiDAR sensor unit 510 and the right LiDAR sensor unit 520 may scan a plane at a predetermined height from the ground, and may detect different parts of the body of the target to be measured by including the first LiDAR sensor units 511, 521 and the second LiDAR sensor units 512, 522, which scan the plane at different heights respectively.

More specifically, the first LiDAR sensor units 511, 521 may collect the first scanning data by scanning a plane at the foot height of the walking target to be measured. That is, the first LiDAR sensor units 511, 521 may detect the foot position, foot shape, and foot direction of the target to be measured by scanning the plane at the foot height of the target to be measured while being attached to the floor or being installed close to the floor. The laser scanning height of the first LiDAR sensor units 511, 521 may be approximately 3 to 10 cm above the ground, more specifically, 5 cm in height.

The second LiDAR sensor units 512, 522 may collect the second scanning data by scanning the plane at the knee height of the walking target to be measured. That is, the second LiDAR sensor units 512, 522 may be installed in the main body 100 in order to detect the knee position of the target to be measured and the direction in which the knee is directed. The laser scanning height of the second LiDAR sensor units 512, 522 may be approximately 20 to 50 cm above the ground and may be configured to be adjustable in height. In this case, the rotary LiDAR may be installed such that the second LiDAR sensor unit 512 of the left LiDAR sensor unit 510 rotates in the first direction and the second LiDAR sensor unit 522 of the right LiDAR sensor unit 520 rotates in the second direction.

Herein, the first LiDAR sensor units 511, 521 and the second LiDAR sensor units 512, 522 may be installed to protrude in the shape of "Y" from the main body 100 as shown in FIGS. 3 and 5. In addition, the front LiDAR sensor unit 300 may be configured to scan the pelvis height of the target to be measured and identify pelvic distortion from the position and direction of the pelvis. The laser scanning height of the front LiDAR sensor unit 300 may be 50 to 100 cm above the ground, and may be configured to be adjustable in height.

The height adjustment unit (not shown) may be a configuration for adjusting the height of the second LiDAR sensor units 512, 522 and the front LiDAR sensor unit 300, and may be composed of a rail and the like. By adjusting the height of the second LiDAR sensor units 512, 522 and the front LiDAR sensor unit 300 to match the height of the knee, the pelvis, and the like of the target to be measured using the height adjustment unit (not shown), it may be possible to accurately measure the gait of the target having different heights to be measured, such as an adult or a child.

The gait analysis unit 600 may analyze at least one selected from the group consisting of body distortion and walking instability when the target to be measured walks, by combining the foot position, knee position, and torso position of the target to be measured. First, the gait analysis unit 600 may specify the foot position of the target to be measured by analyzing the first scanning data, the knee position of the target to be measured by analyzing the second scanning data, and the torso position of the target to be measured by analyzing the scanning data of the front LiDAR sensor unit 300, and may analyze at least one selected from the group consisting of the stride length, the number of the steps, and the walking speed by using the foot position of the target to be measured, and analyze at least one selected from the group consisting of the distance between the knees and the movement speed of the knee by using the knee position of the target to be measured.

In addition, the gait analysis unit 600 may analyze at least one selected from the group consisting of body distortion and walking instability when the target to be measured walks, by combining the foot position, knee position, and torso position of the specified target to be measured. In this way, by analyzing the movement patterns of the torso, the knees, and the feet when walking, it may be possible to provide information necessary for rehabilitation medicine, such as shifts in the body's center of gravity, and to be applied to the cases such as elderly people walking with a cane, individuals with scoliosis, and changes in prognosis before and after surgery.

As described above, according to the mobile robot 10 for gait analysis using the rotary LiDAR proposed by the present disclosure, it may be possible to perform gait analysis simply and easily without attaching a sensor to the body of the target to be measured and to continuously analyze the gait without restrictions on places since tracking and measuring the target to be measured, by measuring the foot movements of the target to be measured and by analyzing the gait using the gait measurement LiDAR sensor unit 500 including at least two rotary LiDARs which are installed to protrude forward from the side of the main body 100, while following the target to be measured with a preset distance maintained by using the scanning information of the front LiDAR sensor unit 300 mounted on the main body 100.

In addition, according to the mobile robot 10 for gait analysis using the rotary LiDAR proposed by the present disclosure, it may be possible to detect the same object at the same time by allowing two rotary LiDARs to rotate in a direction different from each other and thus to accurately track the foot movements of the fast-walking target to be measured, by including the left LiDAR sensor unit 510 where the rotary LiDAR is installed to protrude forward from the left side of the main body 100 in order to rotate in the first direction and the right LiDAR sensor unit 520 where the rotary LiDAR is installed upside down to protrude forward from the right side of the main body 100 in order to rotate in the second direction opposite to the first direction.

Meanwhile, the present disclosure may include a computer-readable medium including a program command for performing operations implemented in various communication terminals. For example, computer-readable media may include magnetic media such as hard disks, floppy disks and magnetic tapes, optical media such as CD-ROMs and DVDs, magnetooptical media such as floptical disks, and hardware devices specifically configured to store and execute program commands such as ROMs, RAMs, flash memories, etc.

Such computer-readable media may include program instructions, data files, data structures, and the like, alone or in combination. In this case, a program command recorded on computer-readable media may be specifically designed and configured to implement the present disclosure or may be known and available to those skilled in the art of computer software. For example, it may include not only machine language codes such as those created by a compiler, but also advanced language codes that may be executed by a computer using an interpreter and the like.

The description of the present disclosure described above is for illustrative purposes only, and those skilled in the art to which the present disclosure belongs will understand that the present disclosure can be easily modified into other specific forms without changing the technical idea or essential features of the present disclosure. Therefore, it should be understood that the exemplary embodiments described above are exemplary in all respects and not limited. For example, each component described in a single form may be implemented in a distributed manner, and similarly, components described as distributed may also be implemented in a combined form.

The scope of the present disclosure is represented by the claims described below rather than the detailed description above, and all modifications or variations derived from the meaning and scope of the claims and the equivalent concept shall be construed as being included in the scope of the present disclosure.

## Claims

1. A mobile robot for gait analysis using a rotary LiDAR, the robot comprising:
a main body;
a driving unit for moving the main body;
a front LiDAR sensor unit mounted on the main body to scan a front side;
a controller for controlling the driving unit to follow a target to be measured with a preset distance maintained, by using scanning information of the front LiDAR sensor unit;
a gait measurement LiDAR sensor unit being installed to protrude forward from a side of the main body and being composed of at least two rotary LiDARs to measure the foot movements of the target to be measured; and
a gait analysis unit for analyzing a gait of the target to be measured, by using the foot movements measured by the gait measurement LiDAR sensor unit,
wherein the gait measurement LiDAR sensor unit comprises:
a left LiDAR sensor unit where a rotary LiDAR is installed to protrude forward from a left side of the main body in order to rotate in a first direction; and
a right LiDAR sensor unit where the rotary LiDAR is installed to protrude forward from a right side of the main body in order to rotate in a second direction opposite to the first direction.

2. The robot of claim 1, wherein the right LiDAR sensor unit allows the rotary LiDAR to be installed upside down to rotate in the second direction.

3. The robot of claim 1, wherein the controller controls the driving unit such that a distance from the left LiDAR sensor unit to the target to be measured is the same as a distance from the right LiDAR sensor unit to the target to be measured.

4. The robot of claim 1, wherein the front LiDAR sensor unit is installed at a torso height of the target to be measured.

5. The robot of claim 1, wherein the gait measurement LiDAR sensor unit measures the foot movements, including changes in position, direction, and shape of the left and right feet, by scanning a plane at a foot height of the target to be measured.

6. The robot of claim 5, wherein each of the left LiDAR sensor unit and the right LiDAR sensor unit comprises:
a first LiDAR sensor unit for collecting a first scanning data by scanning the plane at the foot height of the target to be measured; and
a second LiDAR sensor unit for collecting a second scanning data by scanning a plane at a knee height of the target to be measured.

7. The robot of claim 6, wherein the gait analysis unit analyzes at least one selected from a group consisting of body distortion and gait instability when the target to be measured walks, by combining foot position, knee position, and torso position of the target to be measured.
